# EUROPEAN PATENT SPECIFICATION

(11) **EP 1 882 735 B1**
(45) Date of publication and mention of the grant of the patent: **21.01.2009**
(21) Application number: 06124799.5
(22) Date of filing: 27.11.2006
(51) Int. Cl.: C12M 3/00

(54) **Material sucking or discharging apparatus and method for cleansing the injection needle**
Vorrichtung und Verfahren zum Ansaugen oder Abgeben von Material sowie Verfahren zur Reinigung der Injektionsnadel
Appareil et procédé d'aspiration ou de décharge de matière et procédé de nettoyage d'une aiguille d'injection

(30) Priority: 24.07.2006 JP 2006201013
(43) Date of publication of application: 30.01.2008
(73) Proprietor: FUJITSU LIMITED, Kawasaki-shi, Kanagawa 211-8588 (JP)
(72) Inventor: Shusaku, Nishiyama Fujitsu Limited, Kawasaki-shi Kanagawa 211-8588 (JP)
(74) Representative: Seeger, Wolfgang

(56) References cited:
- EP-A- 0 798 397
- DE-A1- 19 721 084
- JP-A- 1 112 976
- JP-A- 2000 039 467
- US-A1- 2003 228 695
- DATABASE WPI Week 200258 Derwent Publications Ltd., London, GB; AN 2002-543037 XP002459685 & JP 2002 176076 A (NPS KK) 21 June 2002 (2002-06-21)

## Description

The present invention relates generally to a method and apparatus of sucking or discharging a material using an injection needle, a perforated container, and a catheter, which has a fine pore, such as an injection hole and an attraction port. The present invention is suitable, for example, for an injection apparatus or a microinjection apparatus that injects a material, such as gene and medication, into a micro object, such as a cell and colloid, via a capillary.

In the medical applications, there have recently increased opportunities of using cells, to which an injection material, such as gene and medication, has been injected. Unlike the research application, this medical application needs to predetermine a pair of a cell and an injection material and to independently evaluate each cell, *e*.*g*., observe whether or not there is an effect expression in a single cell. It is thus necessary to inject a material into a large amount of cells with a good throughput and injection success rate.

An automatic injection apparatus is necessary for the improved throughput. It is necessary for the improved injection success rate to stably discharge the injection material form the capillary and to stably fix the cell in the container in addition to use the injection method that is universally applied for the artificial inseminations. The injection becomes difficult when the cell freely moves in the laboratory dish. Accordingly, there is proposed a method of simultaneously attracting and fixing many cells on a film with fine pores from the back of the film. See, for example, Japanese Patent Applications, Publication Nos. 2006-149226 and 2-117380.

Other prior art include, for example, Japanese Patent Applications, Publication Nos. 11-1835222 and 2004-166653.

The capillary's internal diameter is below Φ1µm and attracts air bubbles in filling the material. Thus, the material cannot be sometimes expelled even with the pressure due to the damper actions of the air bubbles. The capillary with a dirty tip has difficulties in discharging the material. In producing the capillary, a needle tip contaminates. Moreover, when the material is injected into the cell, parts of the material and cell adhere to the capillary's tip both at the outer and inner parts of the tip. These contaminants and the adhered materials block the injection hole of the capillary, causing the next injection to be insufficient or unsuccessful. In addition, the attraction ports for the fine objects tend to be water-repellent with time, and have difficulties in attracting the floating objects sufficiently.

The present invention is directed to an apparatus and method of stably discharging or sucking a material.

An apparatus according to one aspect of the present invention includes an injection needle and a perforated container that has a pore used to discharge or suck a material, a cleansing unit that irradiates a plasma activated cleansing gas to the injection needle and the perforated container, and a moving unit that moves at least one of the injection needle and the perforated container and the cleansing unit. This apparatus has the cleansing unit, and can provide cleansing just before discharging or sucking of the material. If the injection needle and the perforated container are merely cleansed previously, e.g., at the shipping time from the factory, the cleansing effect for improving the sterilization, disinfection, and wetting property, may be possibly lost just before discharging or sucking. The cleansing effect stabilizes discharging or sucking of the material. Automatic, not manual, cleansing associates with the movement of the moving unit, thereby mitigating the operator's load. A previous confirmation of the cleansing effect would eliminate uneven cleansing that could occur in the manual cleansing, and enhance the cleansing reliability. The type of the material is not specifically limited. The moving unit may move the injection needle and the perforated container and/or the cleansing unit. The apparatus is, for example, an injection apparatus that inserts an injection needle into a fine object, and injects the material into the fine object through the injection needle. The hole may be an injection hole, an attraction (or suction) port, and another pore. The injection needle and the perforated container may conduct both discharging and sucking.

Preferably, the apparatus further includes a decompression device that applies a negative pressure to the inside of the injection needle and the perforated container. This configuration can exert the cleansing effects, such as disinfection, sterilization, and wetting property, on the inner surface of the injection needle and the perforated container. As a result, the influence of the air bubbles and clogs of the material can be reduced or eliminated. In that case, the injection needle and the perforated container may have plural pores, and the decompression device may apply independent and separate negative pressures to the plural pores. This configuration facilitates the attraction of the active cleansing gas.

The apparatus may further include a pressure control mechanism that includes a decompression device that applies a negative pressure to the inside of the injection needle and the perforated container, a pressure device that applies a positive pressure to the inside of the injection needle and the perforated container, and a switch that switches between the decompression device and the pressure device, and a controller that controls a switching action by the switch depending upon cleansing time of the injection needle and operating time of the injection needle and the perforated container. The decompression device and the pressure device share the switch, reducing the number of components, and automatic switching mitigates the operator's load. In that case, the perforated member may have plural pores, and the pressure controller may apply independent and separate pressures to the plural pores. This configuration facilitates the attraction of the active cleansing gas.

Preferably, the apparatus may further include a controller that controls a distance between the pore or the injection needle and the perforated container and an irradiation part of the cleansing gas based on temperature information of the cleansing gas. The temperature information of the cleansing gas is, for example, a temperature distribution in the irradiation range of the cleansing gas. In that case, the apparatus may further include a detector that detects a temperature of the cleansing gas, and informs the temperature information to the controller.

The cleansing unit includes, for example, an oxygen supply source. The cleansing unit may further include an inert gas supply source.

A method according to another aspect of the present invention includes the steps of discharging or sucking a material through a pore in an injection needle and a perforated container, and cleansing the injection needle and the perforated container just before the discharging or sucking step, wherein the cleansing step includes the steps of irradiating a plasma activated cleansing gas to the perforated member, and moving at least one of the injection needle and the perforated container and an irradiation part of the cleansing gas. This method provides cleansing just before discharging or sucking of the material. If the injection needle and the perforated container are merely cleansed previously, e.g., at the shipping time from the factory, the cleansing effect for improving the sterilization, disinfection, and wetting property, may be possibly lost just before discharging or sucking. The cleansing effect stabilizes discharging or sucking of the material. Automatic, not manual, cleansing associates with the moving step, thereby mitigating the operator's load. A previous confirmation of the cleansing effect would eliminate uneven cleansing that could occur in the manual cleansing, and enhance the cleansing reliability. The moving step may move the injection needle and the perforated container and/or the cleansing unit.

The discharging or sucking step may be the step of inserting an injection needle into a fine object and injecting the material into the fine object. Alternatively, the method inserts an injection needle into a fine object held on an attraction port in a container and injecting the material into the fine object through the injection needle, the discharging or sucking step is the step of attracting the fine object into the attraction port in the container.

The method may further include the step of determining whether an order of the injecting step is received, wherein the method automatically executes the irradiating step and the moving step when the determining step determines that the order has been received, thereby automating the cleansing.

The cleansing step may further include the step of generating a negative pressure inside the injection needle and the perforated container during cleansing of the perforated member. This configuration attracts the active cleansing gas into the inner surface of the injection needle and the perforated container aggressively, and can exert the cleansing effects, such as disinfection, sterilization, and wetting property, on the inner surface of the injection needle and the perforated container. As a result, the influence of the air bubbles and clogs of the material can be reduced or eliminated. The cleansing step may further include the step of generating a positive pressure inside the injection needle and the perforated container during cleansing of the injection needle and the perforated container. The positive pressure is particularly effective when the material clogs the perforation.

The method may further include the step of controlling a distance between the pore or the injection needle and the perforated container and an irradiation part of the cleansing gas based on temperature information of the cleansing gas. This configuration can prevent deformations and damages of the injection needle and the perforated container due to the plasma heat. In that case, the method may further include the step of detecting a temperature of the cleansing gas, and the controlling step controls the distance based on a detection result of the detecting step.

The method may further include the step of aligning an irradiation direction of the cleansing gas with a longitudinal direction of the pore or a direction parallel to the longitudinal direction of the pore. This configuration can facilitate the attraction of the cleansing gas into the internal surface of the injection needle and the perforated container.

Other objects and further features of the present invention will become readily apparent from the following description of the preferred embodiments with reference to accompanying drawings.

FIG. 1 is a schematic sectional view of an injection apparatus according to one embodiment of the present invention.

FIG. 2 is a schematic sectional view for explaining a pressure control mechanism for a capillary shown in FIG. 1.

FIG. 3 is a schematic sectional view of a detailed structure near a laboratory dish in the injection apparatus shown in FIG. 1.

FIGs. 4A to 4C are transparent plane views of different channels formed in the dish shown in FIG. 3.

FIGs. 5A and 5B are schematic block diagrams showing examples of the cleansing means applicable to the injection apparatus shown in FIG. 1 or 3.

FIGs. 6A to 6C are schematic perspective views showing illustrative temperature detecting systems applicable to the injection apparatus shown in FIG. 1 or 3.

FIG. 7 is a schematic sectional view of a variation of the injection apparatus shown in FIG. 1.

FIG. 8 is a flowchart for explaining a cleansing method according to one embodiment of the present invention.

FIG. 9 is a flowchart for explaining the details of the cleansing condition determinating step shown in FIG. 8.

FIG. 10 is a flowchart for explaining the details of the cleansing step shown in FIG. 8.

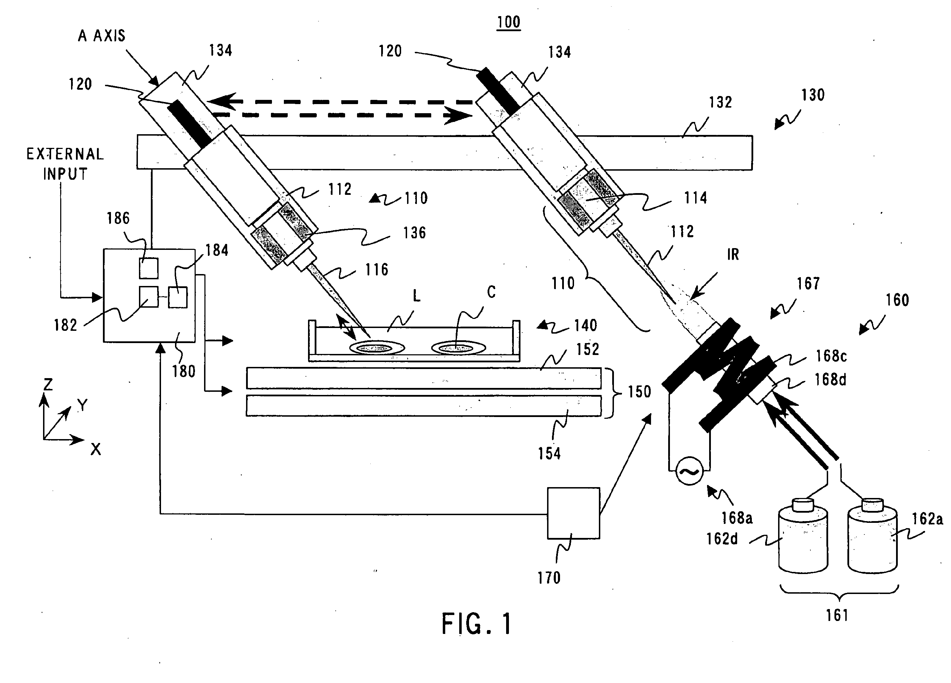

A description will now be given of an injection apparatus 100 according to one embodiment of the present invention, with reference to the accompanying drawings. The injection apparatus 100 injects a predetermined material into a micro object, such as a cell and a colloid, using a capillary. The micro object spreads and can float in a fluid or liquid L. The fluid L is, for example, cell suspension and medium. Here, FIG. 1 is a schematic sectional view of the injection apparatus 100.

The injection apparatus 100 includes, as shown in FIG. 1, a capillary 110, a pressure control mechanism 120, a capillary driving system 130, a laboratory dish 140, a dish driving system 150, a cleansing means 160, a temperature detecting system 170, a control system 180, and an observation system (not shown).

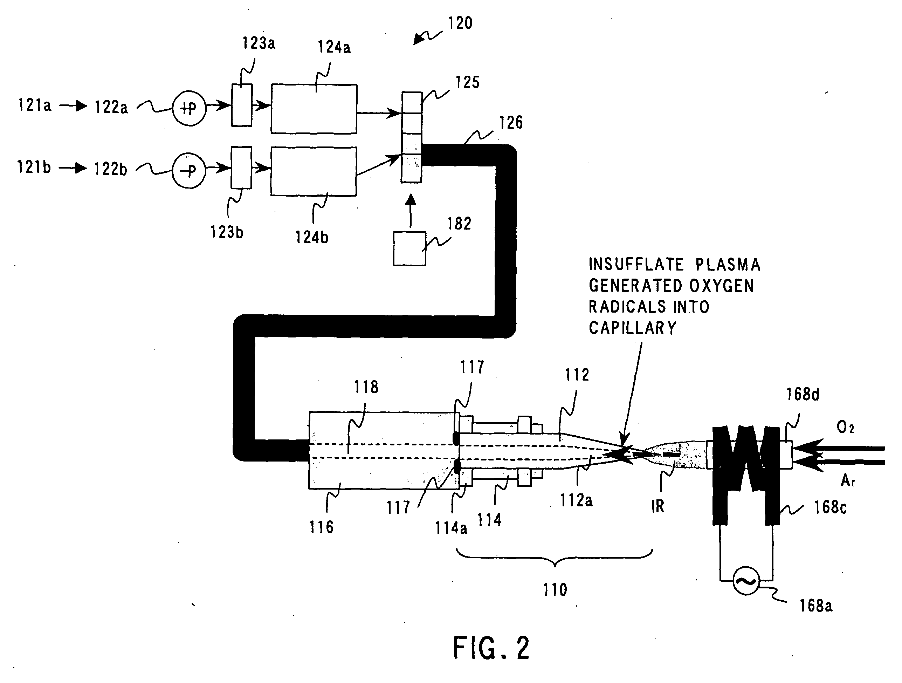

The capillary 110 is an injection needle that is inserted into a specific region in the cell C, such as a nucleus and a cytoplasm, and injects a predetermined material, such as medication and protein, to the specific region. The capillary 110 is connectable to a loader nozzle 115, which will be described later, a plastic hollow manipulator member 116, and the pressure control mechanism 120. The capillary 110 includes, as shown in FIGs. 1 and 2, a glass injection needle 112 having an injection hole 112a, and a plastic holder 114 that holds the injection needle 112.

The holder 114 has an I-shaped section, and includes a flange 114a that is connected to an O-ring 117 at one end of the manipulator member 116 and fixes and seals the injection needle 112 in its axial direction. The loader nozzle 115 is a glass tube that can be inserted into the capillary 110, and fills a predetermined material supplied from material filling means (not shown). A pressure tube 126 of the pressure control mechanism 120 is fixed onto the other end of the manipulator member 116. The manipulator member 116 is made of plastic, and has a channel 118.

The pressure control mechanism 120 controls the pressure in the capillary 110 via the manipulator member 116. The pressure control mechanism 120 includes pressure means 121a and decompression means 121b. The pressure means 121 a includes a pump 122a for the positive pressure, a pressure control valve 123a that maintains the positive pressure constant, a tank 124a that applies a discharging positive pressure, a three-way selector valve 125, and a pressure tube 126. The decompression means 121b includes a pump 122b for the negative pressure, a pressure control valve 123b that maintains the negative pressure constant, a tank 124b that applies a sucking positive pressure, and a three-way selector valve 125. The pressure control mechanism 120 can apply any structure known in the art to each component, and a detailed description thereof will be omitted.

The three-way selector valve 125 is commonly used for the pressure means 121a and the decompression means 121b, and contributes to a reduction of the number of components. The three-way selector valve 125 selects one of the pressure means 121a and the decompression means 121b. The controller 182 in the control system 180 controls the selection by the three-way selector valve 125 so as to select the pressure means 121a in injecting the injection needle 112, and the decompression means 121b in cleansing the injection needle 112. This automatic switching mitigates the operator's load.

The pressure tube 126 is connected to the three-way selector valve 125 at its one end, and to the channel 118 in the manipulator member 116 at its other end. The channel 118 is connected to the injection hole 112a in the injection needle 112. The pressure tube 126 transmits the pressure of the pressure means 121a or the decompression means 121b to the channel 118 selected by the three-way selector valve 125.

The pressure means 121a is used to cleanse the injection needle 112 clogged with a material and/or cell and to inject the material.

The decompression means 121b enables the mixture gas radicals, which will be described later, to be attracted into the injection needle 112, preferably, into the whole internal surface of the injection hole 112a of the injection needle 112 just after the O-ring 117 shown in FIG. 2. This configuration can exert the cleansing effects, such as disinfection, sterilization, and wetting property, not only on the outer surface but also inner surface of the injection needle 112. The disinfection and sterilization effects prevent the contaminations in the subsequent injections. The improved wetting property secures smooth injections of the material.

This embodiment previously sets, through experiments and simulations, the discharge force by which the pressure means 121a injects the material into the cell C without damaging it, and the suction force by which the decompression means 121b attracts the oxygen radicals to the predetermined position.

The capillary 110 is connected to and driven by a capillary driving system 130. At least one of the dish driving system 150 that fixes and supports the dish 140 and the capillary driving system 130 has adjusting means for changing an arrangement and orientation between the capillary 110 and the cells C. The position is, for example, one or more positions in XYZ directions, and the orientation is, for example, one or more angles around each of the XYZ-axes. In this embodiment, the dish driving system 150 has an XY table that serves to move the dish 140 on an XY plane, and the capillary driving system 130 has a moving or fine adjustment function in other directions.

The capillary driving system 130 has first and second moving means, and adjusting means.

The first moving means moves the capillary 110 in a set direction (which is referred to as a "A-axis direction" or "insertion direction" in this embodiment). The first moving means includes a capillary insertion/ejection direct-acting table (A-axis table) 131 that is coupled with the capillary 110 and moves the capillary 110 in the A-axis direction.

The second moving means moves the capillary between the predetermined position and the cleansing position. The predetermined position is not specifically limited, but is an injection ready position above the dish 140 along the A-axis direction or a home position to which the capillary 110 is reset after the injection ends. In FIG. 1, the predetermined position is a left position for the capillary 110, and the cleansing position is a right position for the capillary 110.

The second moving means includes a guide shaft 132 that linearly extends in FIG. 1, a movable part 134 (not shown) that is fixed onto the capillary 110 and movable along the guide shaft 132, and a V-shaped block 136 that fixes the dent of the holder 114 of the capillary 110. The second moving means includes a motor (not shown).

The second moving means may use mechanical means, such as a rack and a pinion fixed onto a motor shaft, magnetic means, such as a linear motor, or other means for the guide shaft 132 and the movable part 134. A movement of the second moving means is a unidirectional linear movement in FIG. 1 along the guide shaft 132, but may be multidirectional linear movements and rotations. The movement mechanism can use any structures known in the art. For example, the second moving means moves the injection needle 112, and then rotates it by a predetermined angle. This rotation would align or parallelize the axial direction of the capillary 110 with the irradiation direction of the mixture gas radicals so as to facilitate the attraction of the mixture gas radicals into the inside of the injection needle 112.

While this embodiment uses the second moving means to moves the capillary 110 to the cleansing position, moving means for moving the cleansing means 160 may be provided to cleanse the capillary 110 at the home position or another position. In addition, both the second moving means and the moving means for the cleansing means 160 may be provided to move both the capillary 110 and the cleansing means 160. In moving the cleansing means 160, it does not have to install the whole cleansing means 160, as described later.

The adjusting means changes an arrangement and orientation between the capillary 110 and the cell C. In an illustration in FIG. 1, the A-axis direction accords with an axial direction of the capillary 110. The adjusting means includes a capillary XY-axes (not shown), a capillary Z-axis (not shown), a β-axis table (not shown), a γ-axis table (not shown), and an α-axis table (not shown). The capillary XY-axes (not shown) provide fine adjustments for the capillary 110 in the XY directions. The capillary Z-axis (not shown) moves the capillary 110 in the Z-axis direction. The β-axis table (not shown) rotates the capillary 110 around the Y-axis or in "β-axis" direction. The γ-axis table (not shown) rotates the capillary 110 around the Z-axis or in "γ-axis" direction. The γ-axis table (not shown) is provided around the dish 140, and has, for example, a hollow disc shape. The α-axis table (not shown) rotates the capillary 110 around the X-axis or in "α-axis" direction.

The dish 140 is a container that houses plural cells C, and has a capturing member 142 (not shown in FIG. 1). However, the cells C adhere to the dish surface when breeding, and it is optional to provide the capturing member 142. The dish 140 is a cylindrical liquid bath that houses the fluid L and the plural cells C that float in the fluid L, and made, for example, of glass or plastic. The dish 140 is replaced with a container that has a channel in another embodiment. The dish 140 is connected to one or more supply parts (not shown) that supply the cells C and the fluid L.

An amount of the fluid L is such that the cells C float in the fluid L without contacting the capturing member 142 during a normal time period and the cells C are restricted on the capturing member 142 during an attraction time period. Since the fluid L flows in the capturing member 142 from attraction ports 143, which will be described later, a sufficient amount of the fluid L needs to be supplied to the dish 140 so that the cells C can float without contacting the capturing member 142 even after the fluid L is absorbed in the capturing member 142.

The dish 140 is provided with a sensor that detects a height of the fluid L. The control system 180 supplies the fluid L to the dish 140 through the supply part (not shown) when the sensor detects that the fluid amount is lower than a predetermined height. The dish 140 may be connected to a channel that feeds the cells into which a predetermined material has been injected (or processed cells) to subsequent recovery part.

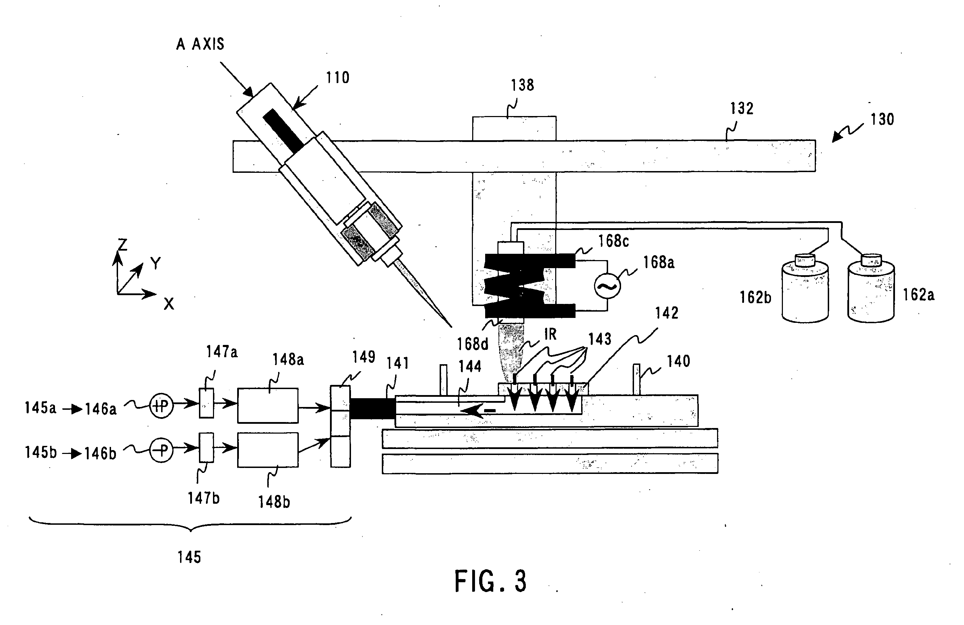

As shown in FIG. 3, the capturing member 142 is a cylindrical member that fits an inner surface of the dish 140, and captures the cells C. The capturing member 142 is made of a material, such as glass and plastic, which has a specific gravity greater than the fluid L and the cells C and does not contaminate the fluid L. The capturing member 142 may be part of the dish 140. Here, FIG. 3 is a schematic sectional view of a detailed structure of the dish 140.

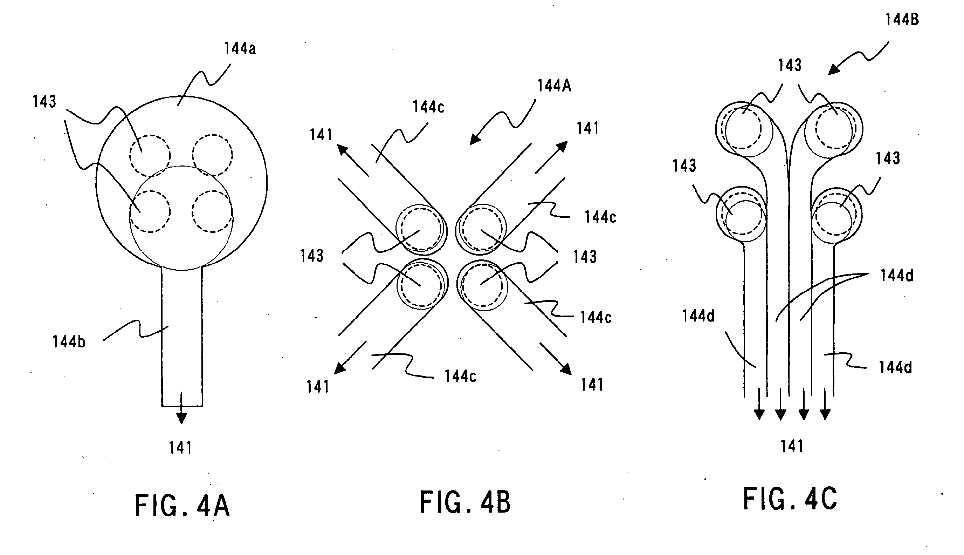

The capturing member 142 has plural (four in FIG. 4) attraction or suction ports 143 having the same size. The attraction ports 143 are holes each having a diameter of several micrometers, and attract and capture the cell C. In this embodiment, centers of four attraction ports 143 correspond to the apexes of the square, but the arrangement of the attraction ports 143 is merely illustrative. The number of attraction ports is set to the maximum number of the cells to be captured simultaneously. The attraction port can attract and exhaust the cell C, exhaust the fluid L, and is connected to the pressure control mechanism 145. A channel 144 is formed in the dish 140, and connected to each attraction port 143.

The channel 144 includes, as shown in FIG. 4A, a hollow disc 144a connected to all the attraction ports 143, and a cylindrical perforation 140b, one end of which is connected to the hollow disc 144a and the other end of which is connected to the pressure control mechanism 145. In this case, the same pressure is simultaneously applied to all the attraction ports 143, and provides no independent and separate pressure control.

A more preferable embodiment replaces the channel 144 with a channel 144A that includes plural cylindrical perforations 140c, as shown in FIG. 4B, one end of which is connected to each attraction port 143, and the other end of which is connected to a different pressure control mechanism 145. This configuration supplies the separate and independent pressure to each attraction port 143, and achieves the independent pressure control, although a pressure control mechanism 145 is necessary for each attraction port 143.

The most preferable embodiment replaces the channel 144 with a channel 144B that includes plural cylindrical perforations 140d, as shown in FIG. 4C, one end of which is connected to each attraction port 143, and the other end of which is connected to the same pressure control mechanism 145 via a control valve (not shown). This configuration supplies the separate and independent pressure to each attraction port 143. The control valve switches the attraction ports 143. Thus, this configuration has an advantage of independent and separate pressure control with only one pressure control mechanism 145 for all the attraction ports 143. The controller 182 controls an operation of the control valve.

The pressure control mechanism 145 includes pressure means 145a and decompression means 145b.

The pressure means 145a includes a pump 146a for the positive pressure, a pressure control valve 147a that maintains the positive pressure constant, a tank 148a that applies a discharging positive pressure, and a three-way selector valve 149. The decompression means 145b includes a pump 146b for the negative pressure, a pressure control valve 147b that maintains the negative pressure constant, a tank 148b that applies an attracting negative pressure, and a three-way selector valve 149. The pressure control mechanism 145 has a structure similar to that of the pressure control mechanism 120, and a detailed description thereof will be omitted.

The three-way selector valve 149 selects one of channels for the pressure means 145a and the decompression means 145b. The controller 182 in the control system 180 controls the selection by the three-way selector valve 149 so as to select the pressure means 145a when the attraction port 143 is to inject, and the decompression means 145b when the attraction port 143 is to attract. This automatic switching mitigates the operator's load.

The pressure tube 141 is connected to the three-way selector valve 125 at its one end, and to the channel 144 at its other end. The channel 144 may be the above channel 144A or 144B. The pressure tube 141 transmits the pressure of the pressure means 145a or the decompression means 145b to the channel 144 selected by the three-way selector valve 149.

The pressure means 145a erupts the liquid L by pressurizing the attraction port 143, thereby releasing the cell C or separating plural aggregating cells C. The pressure means 145a is used to cleanse the attraction port 143 clogged with the material and cell(s).

The decompression means 145b can use the originally equipped decompression means that serves to fix the micro object. The decompression means 145b can attract the mixture gas radicals in the attraction port 143. This configuration can exert the cleansing effects, such as disinfection, sterilization, and wetting property, on the inner surface of the attraction port 143. The disinfection and sterilization effects prevent the contaminations in the subsequent injections. The improved wetting property secures captures of the cell C. When the decompression means 145b affects only each attraction port 143 through the channel 144A or 144B, the mixture gas radicals can be more easily attracted to the desired attraction port 143 than attractions through all the attraction ports 143.

The controller 182 of this embodiment controls the discharge force of the pressure means 145a and the suction force of the decompression means 145b. This embodiment previously sets, through experiments and simulations, the suction force used to capture the cell C without damaging the cell C, the suction force used to attract the oxygen radicals to the predetermined position, and the discharge force used to release the processed cell C. Of course, the suction force used to attract the cell C and the discharge force used to disperse the cells C with liquid L are made adjustable, thereby improving the capturing efficiency.

The dish driving system 150 serves as a means that horizontally fixes and supports the dish 140, and includes an XY table. The XY table has an X-axis table 152 and a Y-axis table 154. The X-axis table 152 moves the dish 140 in a direction parallel to the paper plane of FIG. 1. The Y-axis table 154 moves the dish 140 in a direction perpendicular to the paper plane of FIG. 1. The XY table has a horizontally maintained and fixed disc shape, and roughly positioned on the XY plane by the control system 180. The capillary driving system 130 provides fine adjustments of the capillary 110 and the cells C.

The observation system (not shown) includes a measuring unit that measures a shape of the cell C, and a status observing unit that observes whether the capillary 110 reaches a target position in the cell C, and whether the injection of the material ends. The measuring unit includes an optical system that acquires the cell orientation information, an image processor, an image pickup means, such as a CCD, and is implemented as a measuring unit.

The cleansing means 160 irradiates the plasma activated cleansing gas (mixture gas radicals) to the injection needle 112 and the attraction ports 143 in the dish 140. Use of common cleansing means would reduce the number of components, but the cleansing means 160 for cleansing the injection needle 112 is different from the cleansing means for cleansing the attraction ports 143 in another embodiment.

The injection apparatus 100 includes the cleansing means 160, and provides cleansing just before the injection. Although it is conceivable to cleanse the capillary 110 previously, e.g., at the shipping time from the factory, the cleansing effect does not last and the cleansing effect for improving the sterilization, disinfection, and wetting property, may be possibly lost before the first or n-th injection. The cleansing effect stabilizes the injection of the medication.

Similarly, the injection apparatus can cleanse the attraction port 143 before the cell C is attracted. If the capillary 110 is merely cleansed previously, e.g., at the shipping time from the factory, the cleansing effect for improving the sterilization, disinfection, and wetting property, may be possibly lost before the injection. The cleansing effect stabilizes capture of the cell C in the attraction port 143, and thus the subsequent injection.

The automatic, not manual, cleansing of the injection needle 112 associates with the movement of the second moving means, thereby mitigating the operator's load. A previous confirmation of the cleansing effect would eliminate uneven cleansing that could occur in the manual cleansing, and enhance the cleansing reliability. However, the present invention is not limited to the embodiment that moves the injection needle 112, but allows movements of the injection needle 112 and/or the cleansing means 160. A description will be given later of the automatic cleansing of the attraction port 143.

The cleansing means 160 includes gas supply means 161, and plasma generating means 167. When there are plural cleansing means 160, the gas supply means 161 may be shared.

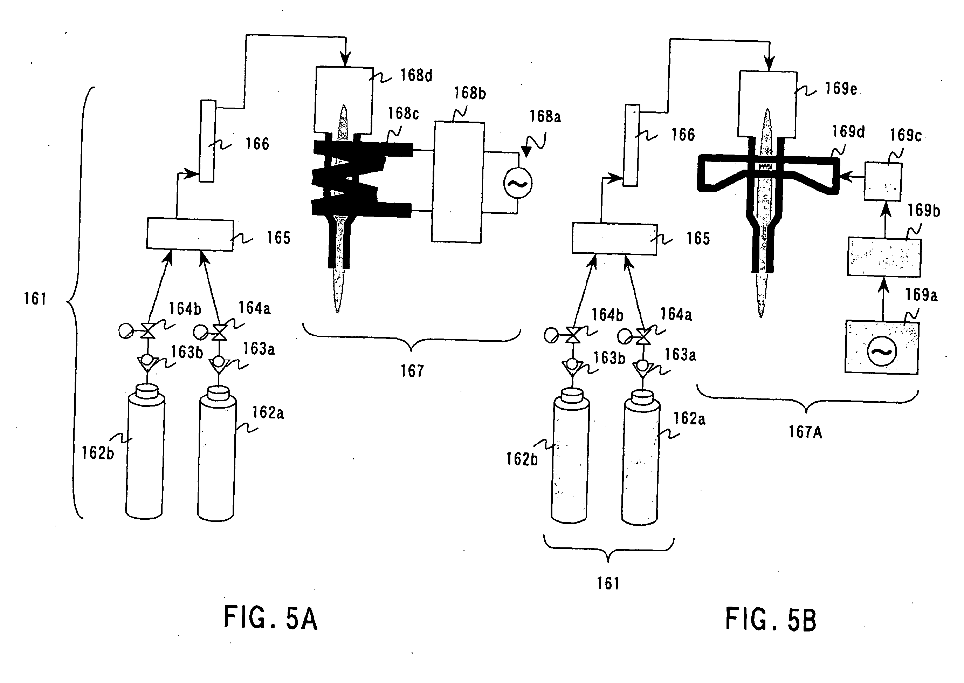

The gas supply means 161 includes, as shown in FIG. 5A, gas supply sources 162a and 162b, check valves 163a and 163b, manometer-cum valves 164a and 164b, a gas mixture 165, and a gas flowmeter 166. The gas supply source 162a stores oxygen gas as a reactive gas, and continuously erupts and supplies the oxygen gas in the arrow direction in FIG. 1. The gas supply source 162b stores argon gas as an inert gas to enhance the plasma efficiency, and continuously erupts and supplies the argon gas in the arrow direction in FIG. 1. A tube may connect the gas flowmeter 166 to the plasma reactor tube 168d.

The reactive gas or process gas becomes active species due to the plasma, and serves to improve cleansing, sterilization, disinfection, and wetting property. The contaminant of the glass material of the injection needle 112 is often polymer, and oxygen radicals are combined with carbon in the polymer into CO₂, providing a degradation effect. Of course, the reactive gas is not limited to oxygen gas, and may use hydrogen and other gases. The inert gas is not limited to argon, and may use other gases, such as neon, xenon, helium, radon, and krypton.

The plasma generating means 167 uses an inductively coupled plasma ("ICP") device. The plasma generating means 167 includes a high frequency power source 168a, a matching box 168b, an inductive coil 168c, and a plasma reactor tube 168d. However, the present invention does not limit the plasma generating means to the ICP source, but may use a microwave-excited surface-wave plasma device shown in FIG. 5B and other plasma generating means. However, the plasma generating means 167 preferably generates the plasma in the air, not in the decompression environment. The plasma generating means 167B shown in FIG. 5B includes a microwave oscillator 169a, a circulator 169b, a directional coupler 169c, a taper waveguide 169d, and a plasma reactor tube 169e. Since the ICP device and microwave-excited surface-wave plasma device may use the conventional structures, a detailed description thereof will be omitted.

FIGs. 1-3, 6 and 7 draw the irradiation range IR of the mixture gas radicals like a flame. This is because oxygen gas and argon gas are ejected in the wind direction indicated by an arrow in FIG. 1. Preferably, this jetting direction is aligned with or parallel to the axial direction of the capillary 110, because the upright orientation of the jetting direction would facilitate the mixture gas radicals into the injection hole 112a.

FIG. 3 fixes the plasma generating means 167 onto the guide shaft 132 of the second moving means via the moving part 138 in the capillary driving system 130. Similar to the guide shaft 132 and the capillary 110's moving part, structures of the guide shaft 132 and the moving part 138 are not limited. The moving part 138 is movable in the horizontal direction along the guide shaft 132 in FIG. 3. Automatic, not manual, cleansing of the attraction ports 143 associates with the movement of the second moving means, thereby mitigating the operator's load. A previous confirmation of the cleansing effect would eliminate uneven cleansing that could occur in the manual cleansing, and enhance the cleansing reliability. However, the present invention is not limited to the embodiment that moves the cleansing means 160, but allows movements of the dish 140 and/or the plasma irradiation part (168b-168d) in the cleansing means 160. FIGs. 1, 3, 6 and 7 omit the matching box 168b.

In order to protect the injection needle 112 and the attraction port 143 from the deformations and damages due to the plasma heat, the injection needle 112 and the attraction port 143 needs to be arranged at a position that does not cause deformations by previously measuring the temperature distribution of the irradiation range IR.

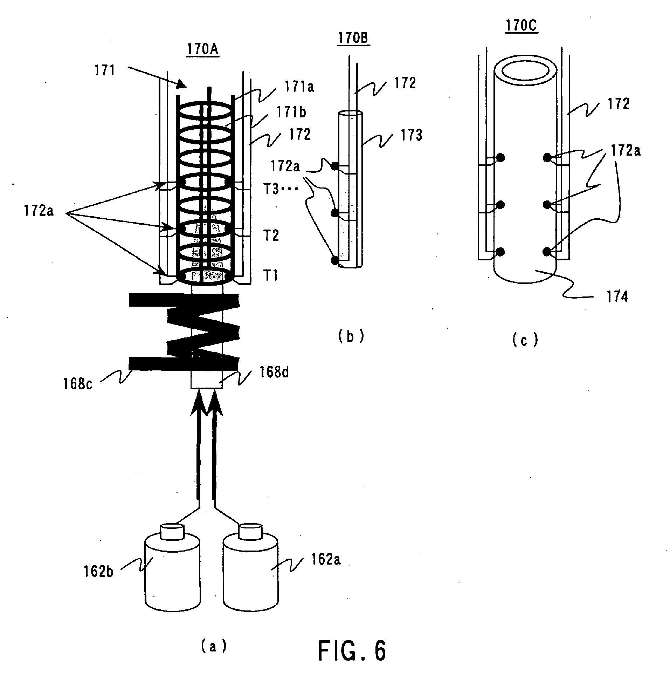

Accordingly, the temperature detection system 170 detects the temperature distribution in the irradiation range IR of the oxygen radicals as the cleansing gas, and informs the temperature information to the controller 182. The temperature detecting system 170 may use, but is not limited to, any one of the temperature detecting system 170A shown in FIG. 6A, the temperature detecting system 170B shown in FIG. 6B, and the temperature detecting system 170C shown in FIG. 6C. FIGs. 6A to 6C are schematic side views of the temperature detecting systems 170A to 170C having differently arranged thermocouples 172a.

The temperature detecting system 170A includes, as shown in FIG. 6A, a structure 171 that has four support shafts 171a that are arranged at four corners of the square, and rings 171b arranged at regular intervals. Plural thermocouples 172a are arranged at symmetrical positions on one ring 171b, and the temperatures T₁, T₂, T₃, ... of the irradiation range IR of the oxygen radicals are detected as averages of the thermocouples 172a fixed onto the same ring 171b. 172 denotes a strand of the sheath type thermocouple.

The temperature detecting system 170B attaches, as shown in FIG. 6B, plural thermocouples 172a to a pipe 173, and forms holes in the pipe 173 at positions corresponding to the thermocouples 172a to expose the thermocouples 172a. The strand 172 is attached to the pipe 173 to fix the strand 172, but the fixture is not necessarily needed as long as the linearity f the strand 172 is maintained.

The temperature detecting system 170C attaches, as shown in FIG. 6C, plural thermocouples 172a to a quartz glass tube 174, and forms holes in the glass tube 174 at positions corresponding to the thermocouples 172a to expose the thermocouples 172a.

The temperature detecting systems 170A and 170B detect the temperature distribution close to that of the actual irradiation range IR, since the mixture gas that has passed the plasma reactor tube 168d spreads in all directions. Since the glass tube 174 defines the channel of the mixture gas radicals, the temperature detecting system 170C would detect the temperature distribution higher than that of the actual irradiation range IR.

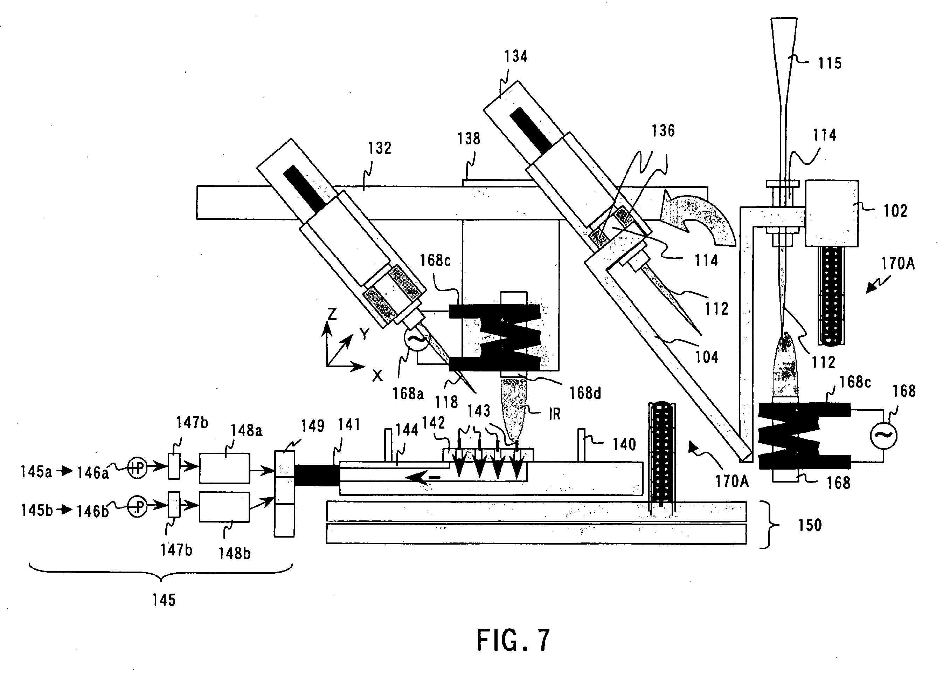

FIG. 7 is a schematic sectional view of the injection apparatus 100A having two cleansing means 160 and two temperature detecting systems 170A. One cleansing means 160 is movably provided near the ready position, and the corresponding temperature detecting system 170A is provided on the injection apparatus frame 102 at the ready position. The plasma generating means 170 in the other cleansing means 160 is mounted on the movable part 138, and the corresponding temperature detecting system 170A is provided on the dish driving system 150. Cleansing is given after the two temperature detecting systems 170A detect the temperature of the irradiation range IR.

The capillary 110 is mounted on an L-shaped support member 104 that can incline around the bottom end. More specifically, the support member 104 holds the holder 114. Thereafter, the capillary is cleansed at the ready position, and then the loader nozzle 115 is inserted, and the injection material is filled. FIG. 7 inserts the loader nozzle 115 into the capillary 110 during cleansing, but the loader nozzle 115 is inserted into the capillary 110 after cleansing. After the injection material is filled, the loader nozzle 115 is removed from the capillary 110. Thereafter, the support member 104 rotates counterclockwise like an arrow around the bottom end. Next, the V-shape block 136 moves from the backside to the front side of the paper, and holds and positions the holder 114 of the capillary 110. Thereafter, the manipulator member 116 moves in the lower right direction, and the O-ring 117 is coupled with the flange 114a.

The control system 140 includes the controller 182, a memory 184, and a timer 186.

The controller 182 calculates an insertion direction and insertion position of the cell C to be inserted into the capillary 110 based on the measurement result by the measuring means in the observation system. Next, the controller 182 controls the first moving means and adjusting means in the capillary driving system 130 and driving of the dish driving system 150 based on the calculated insertion direction and the insertion position.

The controller 182 obtains temperature information of the cleansing gas (mixture gas radicals) from the temperature detecting system 170. The controller 182 controls, based on the temperature information, a distance between the injection needle 112/the attraction port 143 and the irradiating part of the cleansing gas so that the injection needle 112 and the attraction port 143 do not deform or get damaged. If necessary, the controller 182 may control the moving means and the gas flowmeter 166 of the gas supply means 161 in the cleansing means 160 and the high frequency power source 168a of the plasma generating means 167. The controller 182 controls various components of the injection apparatus 100, such as attraction/discharge parts of the cells C, supply part of the liquid L, and injecting part of the material.

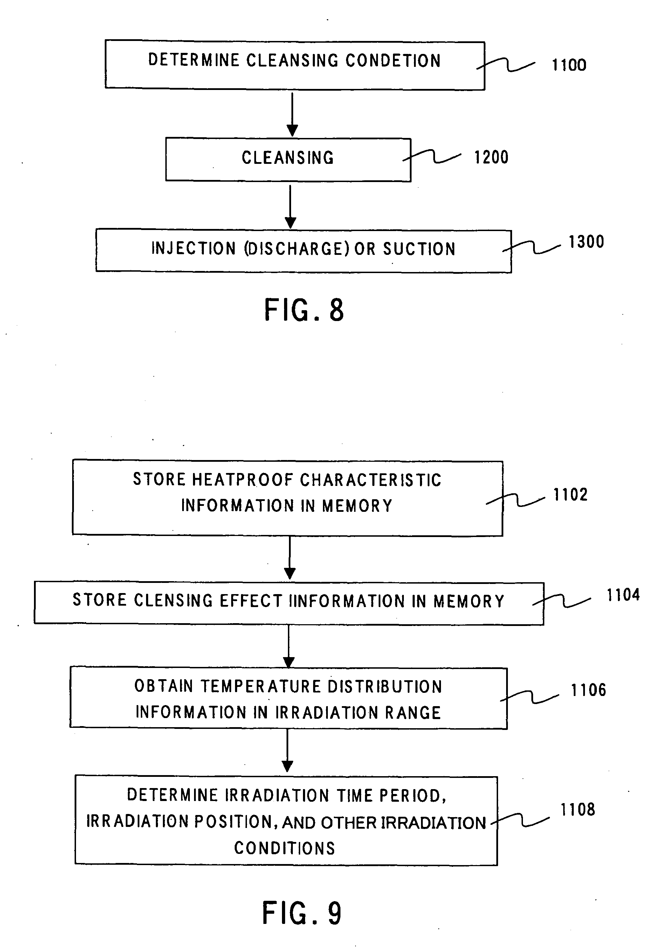

The memory 142 stores operational methods of the controller 182, such as flowcharts shown in FIGs. 8-11, and various data including temperature distribution information of the irradiation range IR, a relationship among the irradiation time period and temperature that does not cause deformations of the injection needle 112 and attraction port 143, the cleansing condition, and the cleansing effect, the flow of the gas flowmeter 166, and the voltage of the high frequency power source 168a.

The timer 186 calculates the predetermined time period, such as a plasma irradiation time period.

A description will be given of the cleansing method of this embodiment with reference to FIGs. 8-10. Here, FIG. 8 is a flowchart showing a cleansing method by the controller 182.

Initially, the controller 182 determines the cleansing condition (step 1100). FIG. 9 shows the details of the step 1100.

First, the controller 182 stores the necessary heatproof characteristic information in the memory 184 (step 1102). The heatproof characteristic information contains information relating to the plasma irradiation temperature and time period that do not cause the deformations of the injection needle 112 and the material around the attraction port 143, such as glass and plastic.

Next, the controller 182 stores necessary cleansing information in the memory 184 (step 1104). The cleansing information contains a cleansing range, cleansing power, and cleansing effect. The cleansing range means, for example, the outer and internal cleansing ranges of the injection needle 112 and the dish 140, the number of attraction ports 143, and the coordinates of the attraction ports 143. The cleansing power has parameters including the negative pressure power, the plasma density, and the cleansing time period. The negative pressure power is one necessary to attract the mixture gas radicals into the predetermined range in the perforated member during cleansing. The plasma density depends upon the voltage of the high frequency power source 168a. The cleansing effect is a demanded effect to improve the sterilization, disinfection, and wetting property. Since the cleansing power usually lowers with the number of uses, the controller 182 may set the cleansing information by considering the number of uses. For example, the cleansing power for the injection needle 112 that has injected 100 times may be made several times as strong as the initial cleansing power.

Next, the controller 182 obtains the temperature distribution information of the irradiation range IR form the temperature detecting system 170 (step 1106). Finally, the controller 182 determines the irradiation time period, the irradiation position, and other irradiation conditions based on the temperature information detected by the temperature detecting system 170 (step 1108). When the controller 182 determines the irradiation position that does not cause the deformations and damages of the perforated member due to the plasma heat, the controller 182 should maintains the intended cleansing effect at that irradiation position. In this case, the controller 182 estimates the plasma density at the irradiation position, and then infers the cleansing power.

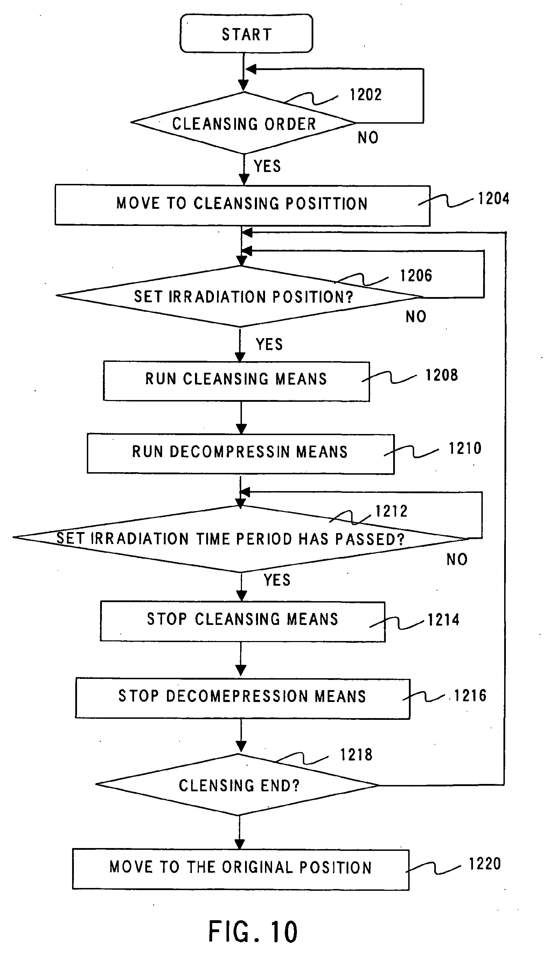

Next, the controller 182 executes cleansing in accordance with the determined cleansing condition (step 1200). FIG. 10 shows the details of the steps 1200.

First, the controller determines whether there is a cleansing order (step 1202). The cleansing order may be an independent cleansing order, or an external input of a projection of the cell suspension, thereby automating cleansing just before the material discharge/attraction.

Next, the controller 182 moves at least one of the perforated member as a cleansing target and the cleansing means 160 via the capillary driving system 130 and the dish driving system 140 (step 1204). The cleansing position is not limited to the right position in FIG. 1. In moving the cleansing means 160, only the irradiating part of the mixture gas radicals as the cleansing gas may be moved as discussed above instead of moving the entire cleansing means 160. In addition, the movement includes the orientation adjustment. In other words, preferably, the irradiation direction of the cleansing gas is aligned with or parallel to the longitudinal direction of the pore of the injection needle and the perforated container, thereby facilitating attractions into the injection needle and the perforated container.

Next, the controller 182 determines whether the injection needle and the perforated container have reached the predetermined irradiation position as a result of that the at least one as the moving target has moved to the predetermined position (step 1006). The controller 182 may determine by directly detecting the perforated member through the detector (not shown) or using the information from the moving means, such as motor's rotating angle.

Next, the controller 182 activates the gas supply means 161 and plasma generating means 167 in the cleansing means 160 (step 1208), and activates the decompression means 121b or 145b (step 1210). The decompression means 145b preferably operates for each attraction port 143. Thereby, cleansing of the perforated member. Since the step 1206 arranges the perforated member at the predetermined position, the injection needle and the perforated container are protected from the deformations and damage due to the plasma heat. This configuration provides automatic, not manual, cleansing after the moving step 1204, mitigating the operator's load. A previous confirmation of the cleansing effect would eliminate uneven cleansing that could occur in the manual cleansing, and enhance the cleansing reliability.

Next, the controller 182 determines whether the set irradiation time period has elapsed based on the time measurement by the timer 186 (step 1212). The controller 182 continues plasma cleansing until it determines that the set irradiation time period has elapsed. When the controller 182 determines that the set irradiation time period has elapsed (step 1212), the controller 182 stops the gas supply means 161 and the plasma generating means 167 in the cleansing means 160 (step 1214), as well as stopping the decompression means 121b or 145b (step 1216).

Next, the controller 182 determines whether cleansing ends (step 1218). For example, when the perforated container is the dish 140, cleansing continues until all the attraction ports 143 are cleansed. Therefore, when the controller 182 determining that cleansing of one attraction port 143 ends returns to the step 1206, and continues the procedure by changing the irradiation position to the adjacent, untreated attraction port 143.

When the controller 182 determines that cleansing is completed (step 1218), the controller 182 moves the at least one to the original position via the capillary driving system 130 and the dish driving system 140 (step 1220).

Turning back to FIG. 8, next follows the injection (discharge) or attraction of the material (step 1300). Thus, this embodiment cleanses the injection needle 112 and the dish 140 that serve as the perforated container (step 1200) just before the discharge or attraction step (step 1300). If the capillary 110 is cleansed previously, e.g., at the shipping time from the factory, the cleansing effect for improving the sterilization, disinfection, and wetting property, may be possibly lost before the injection. The cleansing effect stabilizes the discharge/attraction of the material. For the injection needle 112, the injection (discharge) becomes directly stable. For the attraction port 143, the attraction or fixture of the cell C becomes stable, and thus the injection becomes stable.

The step 1300 fills the material, such as gene and medication, in the capillary 110 when the cleansing target is the injection needle 112: The step 1300 moves and adjust the capillary 110 using the first moving means and the adjusting means, and injects the material into the cell C from the injection needle 112 by controlling the pressure means 121a. The injection becomes stable due to the cleansing. The step 1300 projects the cell suspension into the dish 140 when the perforated container is the dish 140, activates the decompression means 145b, and attracts the cell C. Since the attraction becomes stable due to the cleansing, the subsequent material injection becomes stable.

## Claims

1. An injection apparatus (100) comprising an injection needle (112) and a perforated container (140) that has an injection hole (112a) used to discharge or suck a material, wherein said injection apparatus (100) inserts the injection needle (112) into a fine object (C) and injects the material into the fine object (C) through the injection needle (112),
**characterized in that** said injection apparatus (100) further comprises a cleansing unit (160) that provides a plasma treatment and that irradiates a plasma activated cleansing gas to said injection needle (112), and a moving unit (130) that moves at least one of said injection needle (112) and said cleansing unit (160).

2. An apparatus according to claim 1, wherein the fine object (C) is held on an attraction port (143) in a container (140).

3. An apparatus according to claim 1, further comprising a decompression device (121b, 145b) that applies a negative pressure to the inside of the injection needle (112).

4. An apparatus according to claim 3, wherein the injection needle (112) has plural pores and the decompression device (121b, 145b) applies independent and separate negative pressures to the plural pores.

5. An apparatus according to claim 3, further comprising:
a pressure control mechanism (145) that includes a decompression device (145b) that applies a negative pressure to the inside of the injection needle (112), a pressure device (145a) that applies a positive pressure to the inside of the injection needle (112), and a switch (149) that switches between the decompression device (145b) and the pressure device (145a); and
a controller (182) that controls a switching action by the switch (149) depending upon cleansing time and operating time of the injection needle (112).

6. An apparatus according to claim 5, wherein the injection needle (112) has plural pores, and the pressure controller (145) applies independent and separate pressures to the plural pores.

7. An apparatus according to claim 1, further comprising a controller (182) that controls a distance between the injection hole (112a) or the injection needle (112) and an irradiation part (161) of the cleansing gas based on temperature information of the cleansing gas.

8. An apparatus according to claim 7, further comprising a detector (170) that detects a temperature of the cleansing gas, and informs the temperature information to the controller (182).

9. An apparatus according to claim 1, wherein the cleansing unit (160) includes an oxygen supply source (162a).

10. A method comprising the step of discharging or sucking a material through a pore (112a, 143) in an injection needle (112) and/or in a perforated container (140),
**characterized in that** said method further comprises the step of cleansing the injection needle (112) and/or the perforated container (140) just before said discharging or sucking step, wherein said cleansing step provides a plasma treatment and includes the steps of irradiating a plasma activated cleansing gas to the injection needle (112) and/or the perforated container (140), and moving at least one of the injection needle (112) and/or the perforated container (140) and an irradiation part (161) of the cleansing gas.

11. A method according to claim 10, wherein said discharging or sucking step is the step of inserting an injection needle (112) into a fine object (C) and injecting the material into the fine object (C).

12. A method according to claim 10, wherein said method is a method of inserting an injection needle (112) into a fine object (C) held on an attraction port (143) in a container (140) and injecting the material into the fine object (C) through the injection needle (112), said discharging or sucking step is the step of attracting the fine object (C) into the attraction port (143) in the container (140), said pore being the attraction port (143).

13. A method according to claim 10, further comprising the step of determining whether an order of said injecting step is received, wherein said method automatically executes the irradiating step and said moving step when said determining step determines that the order has been received.

14. A method according to claim 10, wherein said cleansing step further includes the step of generating a negative pressure inside the perforated member (112, 140) during cleansing of the injection needle (112) and/or the perforated container (140).

15. A method according to claim 10, wherein said cleansing step further includes the step of generating a positive pressure inside the injection needle (112) and/or the perforated container (140) during cleansing of the injection needle (112) and/or the perforated container (140).

16. A method according to claim 10, further comprising the step of controlling a distance between the pore (112a, 143) or the injection needle (112) and/or the perforated container (140) and an irradiation part (161) of the cleansing gas based on temperature information of the cleansing gas.

17. A method according to claim 16, further comprising the step of detecting a temperature of the cleansing gas, and said controlling step controls the distance based on a detection result of said detecting step.

18. A method according to claim 10, further comprising the step of aligning an irradiation direction of the cleansing gas with a longitudinal direction of the pore (112a, 143) or a direction parallel to the longitudinal direction of the pore (112a, 143).

## Patentansprüche

1. Injektionsvorrichtung (100), die eine Injektionsnadel (112) und einen perforierten Behälter (140) umfasst, mit einem Injektionsloch (112a), das verwendet wird, um ein Material auszugeben oder anzusaugen, welche Injektionsvorrichtung (100) die Injektionsnadel (112) in ein feines Objekt (C) einführt und das Material durch die Injektionsnadel (112) in das feine Objekt (C) injiziert,
**dadurch gekennzeichnet, dass** die Injektionsvorrichtung (100) ferner eine Reinigungseinheit (160) umfasst, die eine Plasmabehandlung vorsieht und die ein durch Plasma aktiviertes Reinigungsgas auf die Injektionsnadel (112) strahlt, und eine Bewegungseinheit (130), die wenigstens eines von der Injektionsnadel (112) und der Reinigungseinheit (160) bewegt.

2. Vorrichtung nach Anspruch 1, bei der das feine Objekt (C) auf einer Anziehungsöffnung (143) in einem Behälter (140) gehalten wird.

3. Vorrichtung nach Anspruch 1, ferner mit einer Dekomprimierungsanordnung (121b, 145b), die einen Unterdruck auf das Innere der Injektionsnadel (112) anwendet.

4. Vorrichtung nach Anspruch 3, bei der die Injektionsnadel (112) viele Poren hat und die Dekomprimierungsanordnung (121b, 145b) unabhängige und separate Unterdrücke auf die vielen Poren anwendet.

5. Vorrichtung nach Anspruch 3, ferner mit:
einem Drucksteuermechanismus (145), der eine Dekomprimierungsanordnung (145b) enthält, die einen Unterdruck auf das Innere der Injektionsnadel (112) anwendet, eine Druckanordnung (145a), die einen Überdruck auf das Innere der Injektionsnadel (112) anwendet, und einen Schalter (149), der zwischen der Dekomprimierungsanordnung (145b) und der Druckanordnung (145a) umschaltet; und
einem Controller (182), der eine Umschaltaktion durch den Schalter (149) in Abhängigkeit von einer Reinigungszeit und Betriebszeit der Injektionsnadel (112) steuert.

6. Vorrichtung nach Anspruch 5, bei der die Injektionsnadel (112) viele Poren hat und der Druckcontroller (145) unabhängige und separate Drücke auf die vielen Poren anwendet.

7. Vorrichtung nach Anspruch 1, ferner mit einem Controller (182), der eine Distanz zwischen dem Injektionsloch (112a) oder der Injektionsnadel (112) und einem Ausstrahlungsteil (161) des Reinigungsgases auf der Basis der Temperaturinformation des Reinigungsgases steuert.

8. Vorrichtung nach Anspruch 7, ferner mit einem Detektor (170), der eine Temperatur des Reinigungsgases detektiert und die Temperaturinformation dem Controller (182) übermittelt.

9. Vorrichtung nach Anspruch 1, bei der die Reinigungseinheit (160) eine Sauerstoffzufuhrquelle (162a) enthält.

10. Verfahren mit dem Schritt zum Ausgeben oder Ansaugen eines Materials durch eine Pore (112a, 143) in einer Injektionsnadel (112) und/oder in einem perforierten Behälter (140),
**dadurch gekennzeichnet, dass** das Verfahren ferner den Schritt zum Reinigen der Injektionsnadel (112) und/oder des perforierten Behälters (140) gerade vor dem Ausgabe- oder Ansaugschritt umfasst, wobei der Reinigungsschritt eine Plasmabehandlung vorsieht und die Schritte zum Ausstrahlen eines durch Plasma aktivierten Reinigungsgases auf die Injektionsnadel (112) und/oder den perforierten Behälter (140) und zum Bewegen wenigstens eines von der Injektionsnadel (112) und/oder dem perforierten Behälter (140) und einem Ausstrahlungsteil (161) des Reinigungsgases enthält.

11. Verfahren nach Anspruch 10, bei dem der Ausgabe-oder Ansaugschritt der Schritt zum Einführen einer Injektionsnadel (112) in ein feines Objekt (C) und das Injizieren des Materials in das feine Objekt (C) ist.

12. Verfahren nach Anspruch 10, bei dem das Verfahren ein Verfahren ist zum Einführen einer Injektionsnadel (112) in ein feines Objekt (C), das auf einer Anziehungsöffnung (143) in einem Behälter (140) gehalten wird, und zum Injizieren des Materials in das feine Objekt (C) durch die Injektionsnadel (112), der Ausgabe- oder Ansaugschritt der Schritt zum Heranziehen des feinen Objektes (C) an die Anziehungsöffnung (143) in dem Behälter (140) ist, wobei die Pore die Anziehungsöffnung (143) ist.

13. Verfahren nach Anspruch 10, ferner mit dem Schritt zum Bestimmen, ob ein Auftrag für den Injektionsschritt empfangen wurde, wobei das Verfahren den Ausstrahlungsschritt und den Bewegungsschritt automatisch ausführt, wenn bei dem Bestimmungsschritt bestimmt wird, dass der Auftrag empfangen worden ist.

14. Verfahren nach Anspruch 10, bei dem der Reinigungsschritt ferner den Schritt zum Erzeugen eines Unterdrucks im Inneren des perforierten Gliedes (112, 140) während des Reinigens der Injektionsnadel (112) und/oder des perforierten Behälters (140) enthält.

15. Verfahren nach Anspruch 10, bei dem der Reinigungsschritt ferner den Schritt zum Erzeugen eines Überdrucks im Inneren der Injektionsnadel (112) und/oder des perforierten Behälters (140) während des Reinigens der Injektionsnadel (112) und/oder des perforierten Behälters (140) enthält.

16. Verfahren nach Anspruch 10, ferner mit dem Schritt zum Steuern einer Distanz zwischen der Pore (112a, 143) oder der Injektionsnadel (112) und/oder dem perforierten Behälter (140) und einem Ausstrahlungsteil (161) des Reinigungsgases auf der Basis der Temperaturinformation des Reinigungsgases.

17. Verfahren nach Anspruch 16, ferner mit dem Schritt zum Detektieren einer Temperatur des Reinigungsgases, wobei der Steuerschritt die Distanz auf der Basis eines Detektionsresultats des Detektionsschrittes steuert.

18. Verfahren nach Anspruch 10, ferner mit dem Schritt zum Ausrichten einer Ausstrahlungsrichtung des Reinigungsgases mit einer Längsrichtung der Pore (112a, 143) oder einer Richtung, die zu der Längsrichtung der Pore (112a, 143) parallel ist.

## Revendications

1. Appareil d'injection (100) comprenant une aiguille d'injection (112) et un conteneur perforé (140) qui comporte un trou d'injection (112a) utilisé pour décharger ou pour aspirer un matériau, dans lequel ledit appareil d'injection (100) insère l'aiguille d'injection (112) dans un objet fin (C) et injecte le matériau dans l'objet fin (C) à travers l'aiguille d'injection (112),
**caractérisé en ce que** ledit appareil d'injection (100) comprend en outre une unité de nettoyage (160) qui assure à un traitement au plasma et qui irradie un gaz de nettoyage activé par plasma vers ladite aiguille d'injection (112), et une unité de déplacement (130) qui déplace l'un au moins des éléments parmi ladite aiguille d'injection (112) et ladite unité de nettoyage (160).

2. Appareil d'injection selon la revendication 1, dans lequel l'objet fin (C) est tenu sur un orifice d'attraction (143) dans un conteneur (140).

3. Appareil selon la revendication 1, comprenant en outre un dispositif à décompression (121b, 145b) qui applique une pression négative à l'intérieur de ladite aiguille d'injection (112).

4. Appareil selon la revendication 3, dans lequel l'aiguille d'injection (112) comporte une pluralité de pores et le dispositif à décompression (121b, 145b) applique des pressions négatives indépendantes et séparées à la pluralité de pores.

5. Appareil selon la revendication 3, comprenant en outre :
un mécanisme de commande de pression (145) qui inclut un dispositif à décompression (145b) qui applique une pression négative à l'intérieur de l'aiguille d'injection (112), un dispositif à pression (145a) qui applique une pression positive à l'intérieur de l'aiguille d'injection (112), et un commutateur (149) qui commute entre le dispositif à décompression (145b) et le dispositif à pression (145a) ; et
un contrôleur (182) qui commande une action de commutation par le commutateur (149) en dépendance du temps de nettoyage et du temps de fonctionnement de l'aiguille d'injection (112).

6. Appareil selon la revendication 5, dans lequel l'aiguille d'injection (112) possède une pluralité de pores, et le contrôleur de pression (145) applique des pressions indépendantes et séparées à la pluralité de pores.

7. Appareil selon la revendication 1, comprenant encore un contrôleur (182) qui commande une distance entre le trou d'injection (112a) ou l'aiguille d'injection (112) et une partie d'irradiation (161) du gaz de nettoyage en se basant sur les informations de température du gaz de nettoyage.

8. Appareil selon la revendication 7, comprenant encore un détecteur (170) qui détecte une température du gaz de nettoyage, et qui envoie les informations de température au contrôleur (182).

9. Appareil selon la revendication 1, dans lequel l'unité de nettoyage (160) inclut une source de d'alimentation d'oxygène (162a).

10. Procédé comprenant l'étape consistant à décharger ou à aspirer un matériau à travers un pore (112a, 143) dans une aiguille d'injection (112) el/ou dans un conteneur perforé (140),
**caractérisé en ce que** ledit procédé comprend encore l'étape consistant à nettoyer l'aiguille d'injection (112) et/ou le conteneur perforé (140) juste avant ladite étape de décharge ou d'aspiration, dans lequel ladite étape de nettoyage assure un traitement au plasma et inclut les étapes consistant à irradier un gaz de nettoyage activé par plasma vers l'aiguille d'injection (112) et/ou vers le conteneur perfore (140), et à déplacer l'un au moins des éléments parmi l'aiguille d'injection (112) et/ou le conteneur perforé (140) et une partie d'irradiation (161) du gaz de nettoyage.

11. Procédé selon la revendication 10, dans lequel ladite étape de décharge d'aspiration est une étape consistant à insérer une aiguille d'injection (112) dans un objet fin (C) et à injecter le matériau dans l'objet fin (C).

12. Procédé selon la revendication 10, dans lequel ledit procédé est un procédé consistant à insérer une aiguille d'injection (112) dans un objet fin (C) tenu sur un orifice d'attraction (143) dans un conteneur (140) et à injecter le matériau dans l'objet fin (C) à travers l'aiguille d'injection (112), ladite étape de décharge d'aspiration est une étape consistant à attirer l'objet fin (C) dans l'orifice d'attraction (143) dans le conteneur (140), ledit pore étant l'orifice d'attraction (143).

13. Procédé selon la revendication 10, comprenant encore l'étape consistant à déterminer si un ordre concernant ladite étape d'injection est reçu, et dans lequel ledit procédé exécute automatiquement l'étape d'irradiation et ladite étape de déplacement quand ladite étape de détermination détermine que l'ordre a été reçu.

14. Procédé selon la revendication 10, dans lequel ladite étape de nettoyage inclut encore l'étape consistant à générer une pression négative à l'intérieur de l'élément perforé (112, 114) pendant le nettoyage de l'aiguille d'injection (112) et/ou du conteneur perforé (140).

15. Procédé selon la revendication 10, dans lequel ladite étape de nettoyage inclut encore l'étape consistant à la générer une pression positive à l'intérieur de l'aiguille d'injection (112) et/ou du conteneur perforé (140) pendant le nettoyage de l'aiguille d'injection (112) et/ou du conteneur perforé (140).

16. Procédé selon la revendication 10, comprenant encore l'étape consistant à commander une distance entre le pore (112a, 143) ou l'aiguille d'injection (112) et/ou le conteneur perforé (140) et une partie d'irradiation (161) du gaz de nettoyage en se basant sur les informations de température du gaz de nettoyage.

17. Procédé selon la revendication 16, comprenant encore l'étape consistant à détecter une température du gaz de nettoyage, et ladite étape de commande consiste à commander la distance en se basant sur un résultat de détection de ladite étape de détection.

18. Procédé selon la revendication 10, comprenant encore l'étape consistant à aligner une direction d'irradiation du gaz de nettoyage avec une direction longitudinale du pore (112a, 143) ou une direction parallèle à la direction longitudinale du pore (112a, 143).
